# EUROPEAN PATENT SPECIFICATION

(11) **EP 0 764 624 B1**
(45) Date of publication and mention of the grant of the patent: **02.06.1999**
(21) Application number: 95113758.7
(22) Date of filing: 01.09.1995
(51) Int. Cl.: C07C 45/58, C07C 49/167

(54) **Process for the preparation of fluoroketones**
Verfahren zur Herstellung von Fluorketonen
Procédé pour la préparation de fluorocétones

(43) Date of publication of application: 26.03.1997
(73) Proprietor: E.I. DU PONT DE NEMOURS AND COMPANY, Wilmington Delaware 19898 (US)
(72) Inventor: Petrov, Viacheslav Alexandrovich, Wilmington, Delaware 19807 (US); Smart, Bruce Edmund, Wilmington, Delaware 19807 (US)
(74) Representative: Jones, Alan John

(56) References cited:
- US-A- 3 321 515
- US-A- 3 391 119
- US-A- 4 238 416
- US-A- 5 457 238

## Description

This invention concerns a process for the preparation of fluoroketones by the isomerization of the corresponding epoxides in the presence of a Lewis acid catalyst. Fluorinated ketones, such as hexafluoroacetone, are useful intermediates for the synthesis of a variety of fluorinated compounds.

### TECHNICAL BACKGROUND

U.S. Pat. 4,302,608 discloses a continuous process for isomerisation of hexafloropropylene oxide (HFPO) to hexafluoroacetone in the presence of an antimony pentafluoride (SbF₅) catalyst.

U.S. Pat. 3,213,134 discloses the isomerization of epoxide of perfluoroheptene-1 into perfluoroheptanone-2.

U.S. Pat. 3,321,515 teaches that HFPO can be converted into hexafluoroacetone (HFA) by reaction with alumina at 100°C (yield 47%) or by reaction with AlCl₃.

I. L. Knunyants, V. V. Shokina and E. I. Mysov in Izv. AN SSSR. Ser. Khim. 2725 (1973) CA 80, 95151 (1974) disclose the reaction of HFPO with SbCl₅ at 170°C to give a mixture of 15% of chloropentafluoroacetone and 80% of HFA.

A. Ya. Zapevalov, I. P. Kolenko, V. S. Plashkin Zh. Org. Khim. 11,1622 (1975), CA 83, 192954 (1975), and A. Ya. Zapevalov, T. I. Filyakova, M. I. Kodess, I. P. Kolenko Zh. Org. Khim. 22 (1), 93-9 (1986), CA 106, 4771 disclose the use of SbF₅ as catalyst for the isomerization of a number of higher epoxides of polyfluoroolefines but all their examples are limited to fluoroolefins without functional groups because of incompatability of SbF₅ with such groups, for example as -C(O)F. See: T. I. Filyakova, R. E. Ilatovskii, A. Ya. Zapevalov Zh. Org. Khim. 27, NolO, 2055-60 (1991).

Aluminum chloride has limited use in ring-opening reaction of fluoroepoxides because of extensive formation of by-products. See L. A. Saloutina, A. Ya. Zapevalov, M. I. Kodess, I. P. Kolenko and L. S. German Izv. AN SSSR. Ser. Khim. 1434 (1983), CA 99, 157790 z, 1983.

Use of the present process with its aluminum chlorofluoride Lewis acid catalysts improves upon the processes and catalysts dislosed in the art by allowing for better yields with less by-product formation under generally milder temperature and pressure conditions.

### SUMMARY OF THE INVENTION

This invention provides a process for the preparation of fluoroketones of the structure:

R_{f}CF₂C(O)CFXY

wherein X is F or Cl and Y is selected from the group consisting of F, Cl, and R_{f}, wherein R_{f} is F or C₁-C₅ fluoroalkyl, said C₁-C₅ fluoroalkyl optionally containing in-chain oxygen and terminal functional groups; by isomerization of fluorinated epoxides of terminal and internal olefins in the presence of an aluminum chlorofluoride Lewis acid catalyst selected from aluminum chlorofluoride AlFₙCl₃₋ₙ, wherein n is from 0.05-2.95.

The process can be carried out in the optional presence of one or a mixture of inert solvents.

Use of a solid aluminum mixed halide catalyst avoids the handling problems experienced with, for example, SbF₅, which is a viscous, corrosive liquid.

### DETAILED DESCRIPTION OF THE INVENTION

Polyfluorinated ketones R_{f}CF₂C(O)CFXY are prepared by the isomerization of epoxides in the presence of a Lewis acid catalyst, such as AlFₙCl_{3-n'} wherein n is from 0.05 to 2.95, preferably 2.0 to 2.95. Epoxides useful herein are of the formula 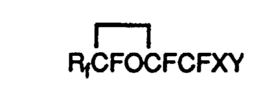 where X is F or Cl and Y is selected from the group consisting of F, Cl, and R_{f}, wherein R_{f} is F or C₁-C₅ fluoroalkyl, said C₁-C₅ fluoroalkyl optionally containing in-chain oxygen and terminal functional groups such as -CN, -OC₆F₅, -C(O)R' (wherein R' is C₁-C₅ alkyl), -SO₂F, -C(O)F. The most preferred epoxide herein is hexafluoropropylene oxide (HFPO).

The reaction is carried out in the presence of an aluminum halide Lewis acid catalyst of the structure AlFₙCl₃₋ₙ wherein n is from 0.05 to 2.95, preferably 2.0 to 2.95. Fluorinated aluminum chloride catalysts can be prepared by the reaction of AlCl₃ and CFCl₃, according to the method described in U.S. 5,162,594, column 4, lines 35-57, which is incorporated herein by reference. Catalyst may be preformed or may be generated in situ. Reactants and catalysts should be free of moisture.

The proportion of catalyst to epoxide is 0.05 to 0.2 mol catalyst per mole of epoxide.

Reaction temperature is about 0-200°C, preferably about 20°C to 100°C. Reaction times can vary from several seconds to about twenty four hours, depending upon temperature, catalyst activity and starting materials.

Solvents are generally not required for the reaction but may, optionally, be used if the solvents are relatively inert to the reaction conditions. By "relatively inert" herein is meant substantially unreactive toward the catalyst at reaction temperatures. Materials suitable for solvents herein are perfluoroalkanes, perfluorocycloalkanes, and perfluoroethers. In some cases, the ketone product of the process can be advantageously used as the solvent.

The process can be carried out in a batch or continuous mode. The reaction can be carried out in the gas phase, in a flow system over a fixed bed catalyst, such as the aluminum chlorofluoride catalyst.

The products of the process are useful intermediates for the synthesis of various fluorinated compounds. For example, HFA is used in the synthesis of bisphenol AF.

### EXAMPLES

### Catalyst preparation, AlCl₃ + CFCl₃

500 g (3.75 mol) of AlCl₃ (Aldrich-99% pure) was stirred mechanically under N₂ in a round bottom flask fitted with a -80°C condenser while 1750 mL (about 2625 g, 19 mol) of CFCl₃ was added over a 1.5 hr period. Reaction is very exothermic in the early stages, so addition of CFCl₃ was slow at first in order to keep the temperature below 65°C, then rapid. The resulting suspension was stirred an additional 3 hrs while volatiles (CF₂Cl₂) were allowed to escape through the condenser. The condenser was then replaced with a simple stillhead, and most of the CCl₄ was distilled under reduced pressure [mainly bp 38°C (266,6 mbar/200 mm)]. Finally, the last traces of volatiles were removed by warming the residual solid to 30-35°C at 0,066 mbar (0.05 mm Hg) pressure.

The sealed round bottom flask was transferred to a dry box and unloaded into a Teflon®FEP bottle; 340 g of rather finely divided yellow-green solid was obtained. Portions of the catalyst were weighed out in the dry box as needed and taken out in plastic bottles with pressure-seal caps.

Analysis for fluorine of the products from preparation of this type indicated the composition to be AlF_{2.9}Cl_{0.1}, AlFₙCl₍₃₋ₙ₎; n = 2.9. The aluminum chlorofluoride catalyst is abbreviated ACF herein.

### EXAMPLE 1

Two g of ACF and 20 mmol of HFPO were loaded into an evacuated cylinder through a vacuum line at -196°C. The cylinder was warmed up to 25°C. After 2 h at this temperature, gas-phase IR confirmed that all HFPO was converted into HFA. The yield was quantitative.

### EXAMPLE 2

Inside of a dry box, 0.3 g of ACF was placed in a 5 mL glass sample tube equipped with Teflon stopcock; 1 g of oxide 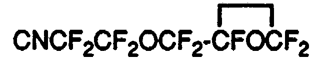 was added in one portion. The tube was closed. Exothermic reaction was observed. After 2 h only the corresponding ketone CNCF₂CF₂OCF₂C(O)CF₃ was found in the sample tube according to ¹⁹F NMR, IR and GC. IR (neat): 2273 (CN), 1807 (C=O) cm⁻¹. The yield was quantitative.

### EXAMPLE 3

As in Example 2, 0.3 g of ACF was used for quantitative isomerization of 1 g of oxide 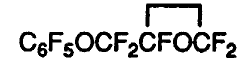 into ketone C₆F₅OCF₂ C(O) CF₃. IR: 1802 (C=O) cm⁻¹, on the ¹⁹F NMR spectrum corresponds to the proposed structure.

### EXAMPLE 4

As in Example 1, a mixture of 0.5 g ACF and 5 g of perfluoro-2,3-epoxypentane (85% purity, the rest perfluoropentene-2) was kept at 150°C for 18 h. According to GC and ¹⁹F NMR data, the reaction mixture contained 37% perfluoro-2-pentanone, 26% perfluoro-3-pentanone, 22% of starting oxide and 15% of perfluoropentene-2. Conversion of oxide was 74%, yield of ketones >95%.

### EXAMPLE 5

Hexafluoropropylene oxide (13.8 g) was bubbled through a suspension of 2 g of ACF suspended in 50 mL of the cyclic dimer of hexafluoropropene at the rate of 0.04 g/min at 25°C and atmospheric pressure. The outcoming gases (19.3 g) were collected in a cold (-78°C) trap protected against atmospheric moisture. The product, according to ¹⁹F NMR data, was a mixture of 68% of hexafluoroacetone and 32% of solvent, no starting material was found. The yield of HFA was 94%, conversion of hexafluoropropylene oxide was 100%.

## Claims

1. A process for the preparation of fluoroketones of the structure:
R_{f}CF₂C(O)CFXY
wherein X is F or Cl and Y is selected from the group consisting of F, Cl, and R_{f}, wherein R_{f} is F or C₁-C₅ fluoroalkyl, said C₁-C₅ fluoroalkyl optionally containing in-chain oxygen and terminal functional groups comprising:
isomerization of fluorinated epoxides of terminal and internal olefins of the structure: 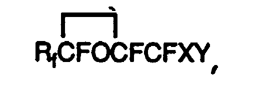 where X, Y and R_{f} are as defined above, in the presence of a Lewis acid catalyst selected from the group consisting of aluminum chlorofluoride AlFₙCl₃₋ₙ, wherein n is from 0.05 to 2.95.

2. The process of Claim 1 wherein n is 2.0 to 2.95.

3. The process of Claim 1 carried out in the presence of an inert solvent.

4. The process of Claim 3 wherein the solvent is selected from the group consisting of perfluoroalkanes, perfluorocycloalkanes, and perfluoroethers.

5. The process of Claim 3 carried out in the fluoroketone product of Claim 1.

6. The process of Claim 1 carried out at a temperature of 0°C to 200°C.

7. The process of Claim 1 carried out at a temperature of 20°C to 100°C.

8. The process of Claim 1 wherein the proportion of catalyst to epoxide is 0.05 to 0.2 mole of catalyst per mole of epoxide.

9. The process of Claim 1 carried out over a bed of solid catalyst.

10. The process of Claim 1 wherein the starting material is HFPO and the product is HFA.

## Patentansprüche

1. Verfahren zur Herstellung von Fluorketonen der Struktur:
R_{f}CF₂C(O)CFXY
wobei X F oder Cl ist und Y ausgewählt ist aus der Gruppe, bestehend aus F, Cl und R_{f}, wobei R_{f} F oder C₁C₅-Fluorölkyl ist, wobei das C₁-C₅-Fluoralkyl gegebenenfalls Sauerstoff in der Kette und endständige funktionelle Gruppen enthält, umfassend:
Isomerisierung fluorierter Epoxide endständiger und innerlicher Olefine der Struktur: 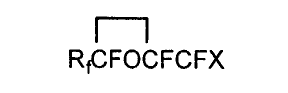 wobei X, Y und R_{f} wie vorstehend definiert sind, in Anwesenheit eines Lewissäurekatalysators, ausgewählt aus der Gruppe, bestehend aus Aluminiumchlorofluorid AlFₙCl₃₋ₙ, wobei n 0,05 bis 2,95 ist.

2. Verfahren nach Anspruch 1, wobei n 2,0 bis 2,95 ist.

3. Verfahren nach Anspruch 1, ausgeführt in Anwesenheit eines inerten Lösungsmittels.

4. Verfahren nach Anspruch 3, wobei das Lösungsmittel aus der Gruppe, bestehend aus Perfluoralkanen, Perfluorcycloalkanen und Perfluorethern, ausgewählt ist.

5. Verfahren nach Anspruch 3, ausgeführt in dem Fluorketonprodukt nach Anspruch 1.

6. Verfahren nach Anspruch 1, ausgeführt bei einer Temperatur von 0°C bis 200°C.

7. Verfahren nach Anspruch 1, ausgeführt bei einer Temperatur von 20°C bis 100°C.

8. Verfahren nach Anspruch 1, wobei das Verhältnis von Katalysator zu Epoxid 0,05 bis 0,2 Mol Katalysator pro Mol Epoxid beträgt.

9. Verfahren nach Anspruch 1, ausgeführt über einem Bett des festen Katalysators.

10. Verfahren nach Anspruch 1, wobei das Ausgangsmaterial HFPO ist und das Produkt HFA ist.

## Revendications

1. Procédé pour la préparation de fluorocétones avec la structure:
R_{f}CF₂C(O)CFXY,
dans laquelle X est F ou Cl et Y est choisi parmi le groupe composé de F, Cl et R_{f}, dans lequel R_{f} est F ou un groupe fluoroalkyle C₁-C₅, ledit groupe fluoroalkyle C₁-C₅ contenant facultativement un oxygène dans une chaîne et des groupes fonctionnels terminaux,
comprenant:
l'isomérisation d'époxydes fluorés d'oléfines terminales et internes avec la structure: 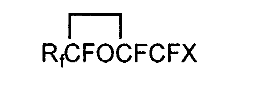 où X, Y et R_{f} sont définis ci-dessus, en présence d'un catalyseur acide de Lewis choisi parmi le groupe composé de chlorofluorure d'aluminium AlFₙCl₃₋ₙ, dans lequel n varie de 0,05 à 2,95.

2. Procédé de la revendication 1, dans lequel n varie de 2,0 à 2,95.

3. Procédé de la revendication 1 réalisé en présence d'un solvant inerte.

4. Procédé de la revendication 3, dans lequel le solvant est choisi parmi le groupe composé de perfluoroalcanes, de perfluorocycloalcanes et de perfluoroéthers.

5. Procédé de la revendication 3 réalisé dans le produit de fluorocétone de la revendication 1.

6. Procédé de la revendication 1 réalisé à une température allant de 0°C à 200°C.

7. Procédé de la revendication 1 réalisé à une température allant de 20°C à 100°C.

8. Procédé de la revendication 1, dans lequel la proportion de catalyseur par rapport à l'époxyde varie de 0,05 à 0,2 moles de catalyseur par mole d'époxyde.

9. Procédé de la revendication 1 réalisé sur un lit de catalyseur solide.

10. Procédé de la revendication 1, dans lequel le matériau de départ est de l'HFPO et le produit est de l'HFA.
